# EUROPEAN PATENT SPECIFICATION

(11) **EP 1 767 644 B1**
(45) Date of publication and mention of the grant of the patent: **13.01.2010**
(21) Application number: 05108759.1
(22) Date of filing: 22.09.2005
(51) Int. Cl.: C12P 13/00, C12N 9/02, C12N 1/14, C12N 1/20

(54) **Microbial strains producing sphingosine**
Mikrobielle Stämme zur Produktion von Sphingosin
Couches microbiénnes produisant sphingosine

(43) Date of publication of application: 28.03.2007
(73) Proprietor: Cosmoferm B.V., 2611 XT Delft (NL)
(72) Inventor: Van den Berg, Marco Alexander, 2685 EH Poeldijk (NL); Streekstra, Hugo, 1017 SP Amsterdam (NL)
(74) Representative: van Heuvel, Margaretha

(56) References cited:
- WO-A-00/01839
- WO-A-95/12683
- AVERET NICOLE ET AL: "Yeast mitochondrial metabolism: From in vitro to in situ quantitative study" MOLECULAR AND CELLULAR BIOCHEMISTRY, vol. 184, no. 1-2, July 1998 (1998-07), pages 67-79, XP002366402 ISSN: 0300-8177

## Description

The present invention relates to microbial strains that are capable of producing the sphingoid base sphingosine.

The term "sphingolipids" refers to a group of lipids that are derived from sphingoid bases like sphingosine. Sphingolipids occur frequently in cellular membranes of animals, plants and microorganisms.

Ceramides are a specific group of sphingolipids containing sphingosine, phytosphingosine or dihydrosphingosine (sphinganine) as a base (sphingoid base) in amide linkage with a fatty acid. Ceramides are the main lipid component of the stratum corneum, the upper layer of the skin. Topical application of compositions comprising sphingolipids, such as ceramides, improves the barrier function and moisture-retaining properties of the skin (Curratolo, 1987, Pharm. Res. 4, 271-277; Kerscher et al., 1991, Eur. J. Dermatol. 1, 39-43). Furthermore, sphingoid bases as such are known to mediate several physiological effects as inhibiting the activity of protein kinase C and are therefore included in cosmetic or dermatological compositions for their anti-inflammatory and antimicrobial activity.

As sphingosine is the major sphingoid base component of sphingolipids in human, it is of considerable commercial interest to produce sphingosine and sphingosine-containing sphingolipids for food, pharmaceutical and cosmetic applications.

Currently, several routes for the chemical synthesis of sphingosine (and sphinganine) have been developed. However, due to the presence of two stereocenters in these molecules, chemical synthesis results in a racemic mixture with only 25% representing the naturally occurring stereochemical configuration. Moreover, extensive protection chemistry has to be applied due to the presence of three functional groups within these molecules. Consequently, sphingosine and sphinganine produced via chemical synthesis are extremely expensive, which does not allow for its incorporation into food and cosmetic formulations. This is also true for pure sphingosine and sphinganine isolated from natural sources, such as brain or chicken eggs. Heterogeneous sphingolipids preparations, which have been extracted from animal sources, are also available. Though cheaper than the pure compounds, they suffer from compositional heterogeneity and are potentially unsafe as they might contain pathogenic agents.

Microorganisms as the yeast *Pichia ciferrii* (Wickerham and Stodola, 1960, J. Bacteriol. 80, 484-491) were shown to produce sphingoid bases and derivatives thereof, but mainly C18-phytosphingosine and acetylated derivatives thereof. These can be extracted and chemically converted into other sphingoid bases and/or ceramides, thereby obtaining pure cosmetic ingredients (see e.g. WO 93/20038).

However, an economically feasible way of producing the naturally occurring stereoisomers of sphingosine and sphinganine as pure compounds is not available and is extremely desirable.

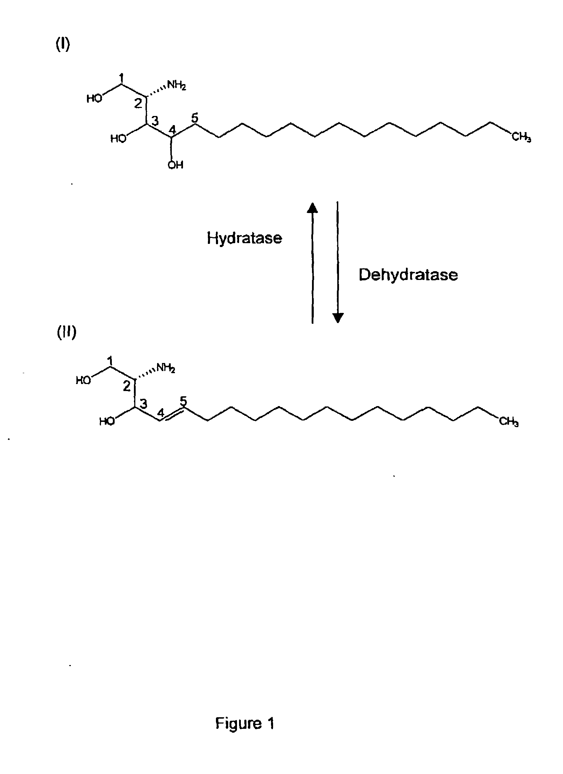

The present invention surprisingly shows the identification of microorganisms that are able to convert phytosphingosine into sphingosine. The ability to perform this conversion is due to the presence in the microorganism of a suitable enzyme activity, i.e. a dehydratase (see Figure 1). The identification of such a dehydratase was not a simple task, as such enzymes often catalyse both forward and reverse reactions and in water (or in acidic conditions) usually hydratation is favored; therefore the equilibrium of the enzymatic reaction lies towards the alcohol (phytosphingosine). Only if the alkene (sphingosine) is extremely stabilized by, for example, phenyl or other conjugating groups or the kinetics of the enzyme favor one direction of the reaction, the equilibrium may lie less extremely towards one side. Chemically, elimination of water only takes place under drastic conditions like concentrated H₂SO₄, gas phase heating over AL₂O₃ or H₃PO₄.

Surprisingly, the present invention provides microbial strains and enzymes capable of dehydratation of phytosphingosine.

In a first aspect, the present invention provides a process to screen a series of candidate microorganisms for their capacity to convert exogenous phytosphingosine into sphingosine and to identify a microorganism that is able to convert at least part of the phytosphingosine into sphingosine.

The process comprises:
providing a series of candidate microorganisms,
incubating said series of candidate microorganisms in the presence of a suitable amount of phytosphingosine,
analyzing the composition of the sphingoid base obtained from each incubated microorganism, and
identifying a microorganism that is able to convert at least part of the phytosphingosine into sphingosine.

The microorganism that is subjected to the process of the invention can be any microorganism, i.e. a bacterium, a yeast or a fungus. Preferably, eukaryotic microorganisms or Actinomycete bacteria are used for the screening. The candidate microorganisms are cultured until a desired cell density is reached, whereupon for each microorganism the cells are harvested, according to procedures known in the art.

If the relevant microbial enzyme(s) accumulate(s) intracellularly, the microbial cells are preferably permeabilised prior to incubation with phytosphingosine, to ensure that the relevant microbial enzyme(s) are in contact with the exogenously added, hydrophobic substrate phytosphingosine. Permeabilisation may be done by any suitable treatment that results in cell wall / membrane permeability. For instance, a suitable amount of microbial cells may be treated with an organic solvent not miscible with water, such as toluene, under vigorous stirring. Treatment typically is done at a pH around neutral, e.g. a pH of 6-8, and a temperature that is similar to the temperature used for culturing the microorganism. Permeabilisation may also be done by using an agent capable of introducing pores in the cell membrane. An example of such an agent is nystatin (Avéret N, Fitton V, Bunoust O, Rigoulet M, Guérin B. 1998. Yeast mitochondrial metabolism: from in vitro to in situ quantitative study. Mol Cell Biochem 184: 67-79). Permeabilisation may also be done by vigorous vortexing without the addition of any specific chemicals. The permeabilising treatment is done until the permeability of the cells reaches a sufficient level. Care should be taken to prevent too far cell permeabilisation, resulting in cell lysis. The extent of permeability may be tested by testing the activity of a suitable intracellular indicator enzyme, for example isocitrate dehydrogenase or by testing whether suitable dyes, e.g. fluorescent dyes, can enter the cells.

The permeabilised cells are then incubated with a suitable amount of phytosphingosine, whereby phytosphingosine preferably is solubilised in an aqueous solution. To solubilise phytosphingosine in aqueous solution, a suitable amount of an alcohol, e.g. ethanol, and/or a surfactant, e.g. Tween-80, is present in the phytosphingosine solution. Ethanol and Tween-80 may be used at concentrations up to 20%. Incubation may be done at a temperature of 10° to 40°C, typically at a temperature that is similar to the culture temperature of the microorganism. The incubation may be for 10 minutes to 24 hours, typically 2 hours, at a pH around neutral. The phytosphingosine concentration in the incubation mixture may be from 0.1 to 100mM, preferably from 1 to 20 mM. After incubation, the obtained reaction product is dried, preferably by lyophilisation, optionally preceded by storage of the liquid reaction product at -70°C.

To identify a microorganism that is able to convert at least part of the exogenously added phytosphingosine into sphingosine, the reaction product of the incubation of (permeabilised) cells with phytosphingosine is analysed for its sphingoid base composition. To this end, the sphingoid base is extracted from the microbial cell residue present in the reaction product and treated to derivatise the sphingoid base.

Prior to extraction of the sphingoid base, the reaction product may be pulverised or milled, as the sample used for sphingoid base extraction and derivatisation should preferably consist of finely divided solids.

Extraction of the sphingoid base is done by vigorous mixing of the reaction product with a suitable organic solvent , e.g. chloroform, methanol and/or ethanol. Any crystals that are present may be removed by e.g. ultrasonic treatment. In a preferred embodiment, derivatisation is done on the sphingoid base as present in the reaction mixture without firstly performing a separation of extracted sphingoid base. More preferably, the derivatisation agent is already present during extraction.

Derivatisation may be done by treatment of the sphingoid base with, for instance, acetic anhydride, trifluoro acetic anydride or o-phthalaldehyde, in a suitable solvent. The treatment may be for 30 minutes at 35°C Depending on the matrix (e.g. microorganism) and specific conditions, optimization of the treatment may be needed. This could result in incubation times ranging from 1 to 240 minutes and temperatures ranging from 20 to 50 °C.

The sphingosine content of the extracted sphingoid base is determined by e.g. HPLC or NMR analysis. Extracted sphingoid base comprises sphingoid bases natively present in the microbial cells as well as, where applicable, sphingosine as produced from exogenously added phytosphingosine.

According to the invention, microorganisms are identified that are able to convert at least part of exogenously added phytosphingosine into sphingosine to such an extent that the sphingosine content of extracted sphingoid base is at least 5%, preferably at least 10%, more preferably at least 15%, most preferably at least 20% (w/w), using phytosphingosine in a concentration of 10 mM. Examples of identified microorganisms are microorganisms from the genus (species) *Pseudomonas (aurantiaca), Grifola (frondosa), Streptomyces, Inonotus (dryophilus), Hericium (coralloides), Phytophthora (capsici), Tricellula (aurantiaca), Pythium (adhaerens).*

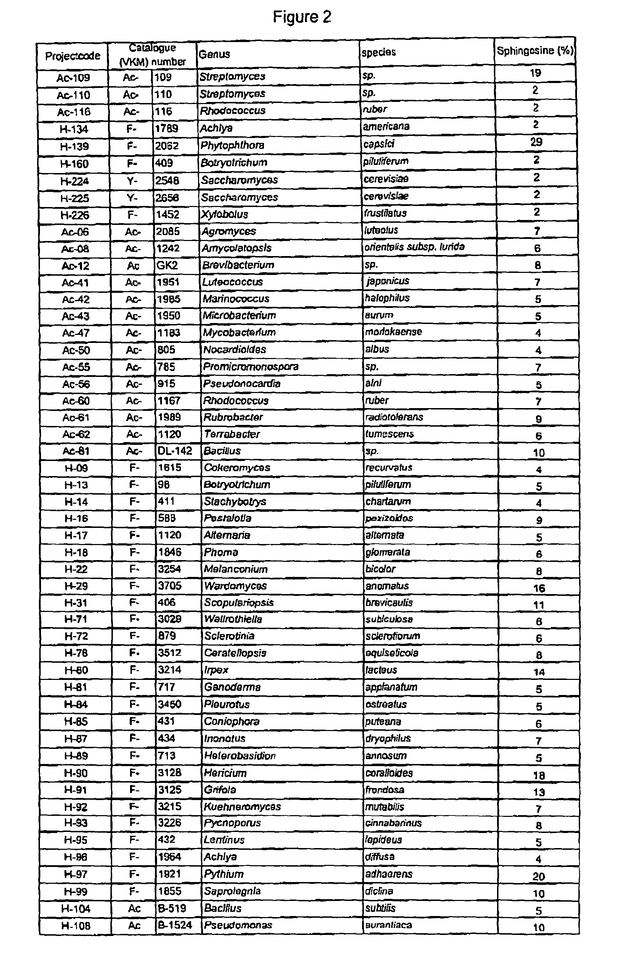

In Figure 2 an overview of identified microorganisms is provided.

In a second aspect, the present invention provides a process for the preparation of sphingosine by cultivating the microorganism as defined in claim 10, incubating the microorganism in the presence of phytosphingosine to convert at least part of the phytosphingosine into sphingosine and recovering the sphingosine.

Incubation of the microorganism in the presence of phytosphingosine may conveniently be done using conditions similar to those mentioned for the identification of microorganisms that are capable of converting phytosphingosine to sphingosine.

Preferably, the phytosphingosine used is obtainable from *Pichia ciferrii.*

The sphingosine obtained after incubation of the microorganisms with phytospingosine can directly be recovered from the total broth, or more preferably after acylation according to methods known in the art for sphingolipid recovery. The acylation can range from mono- to tri-acylated forms of sphingosine. Preferably, the acylation is acetylation.

Classical mutagenesis methods may be used to isolate variants of the phytosphingosine to sphingosine converting microorganisms, e.g. variants that can resist higher concentrations of phytosphingosine and/or perform the conversion at a higher rate.

In a third aspect, the present invention provides for the use of the microorganism as defined in claim 11 to isolate the enzyme activity that converts phytosphingosine into sphingosine. This enzyme activity preferably is a dehydratase activity. In addition, the present invention provides the use of the microorganism as defined in claim 12 to obtain a microorganism that produces sphingosine, by isolating the gene(s) encoding the particular enzyme (dehydratase) activity, transforming the gene(s) into a suitable phytosphingosine-producing microorganism, preferably *Pichia ciferrii*, and isolating a transformed microorganism that produces sphingosine. Such a transformed microorganism can be advantageously used to directly produce sphingosine.

### Legends to the Figures

Figure 1. Phytosphingosine (I) conversion into Sphingosine (II) via a dehydratase enzyme.
Figure 2. Overview dehydratase positive organisms.
Figure 3. Sphingoid base analysis using Acetic Anhydride (AA).
Figure 4. Dehydratase activity by *Pseudomonas aurantiaca* (Nakhimovskaya 1948, VKM B-1524). TFAA-based HPLC analysis of Sphingosine produced from Phytosphingosine. A. direct sample analysis; B. sphingosine spiked to the sample to confirm formation of sphingosine.

### EXAMPLE 1

### Analysis of sphingoid bases via derivatization with acetic anhydride

As a derivatization reagent 600 µl acetic anhydride was mixed with 40 mg 4-(dimethylamino) pyridin and 200 µl triethylamine. Chloroform was added till 10 ml and the solution was mixed on a magnetic stirrer. Two mg of either phytosphingosine or sphingosine were mixed with 250 µl derivatization reagent and sonicated until the crystals are dissolved. The solutions were placed in a 35°C water bath for 30 min, acetonitrile was added up to 5 ml and mixed with a vortex. Ten µl was analysed by HPLC, using the following conditions.

| HPLC conditions | |
|---|---|
| Mobile phase | 0.5 ml trifluoro acetic acid + in 1000 ml acetonitrile |
| Column | GL Science, Inertsil ODS 80-A, 4.6 x 250 mm |
| Flow | 1 ml/min |
| UV-detector Wavelength | 200 nm |

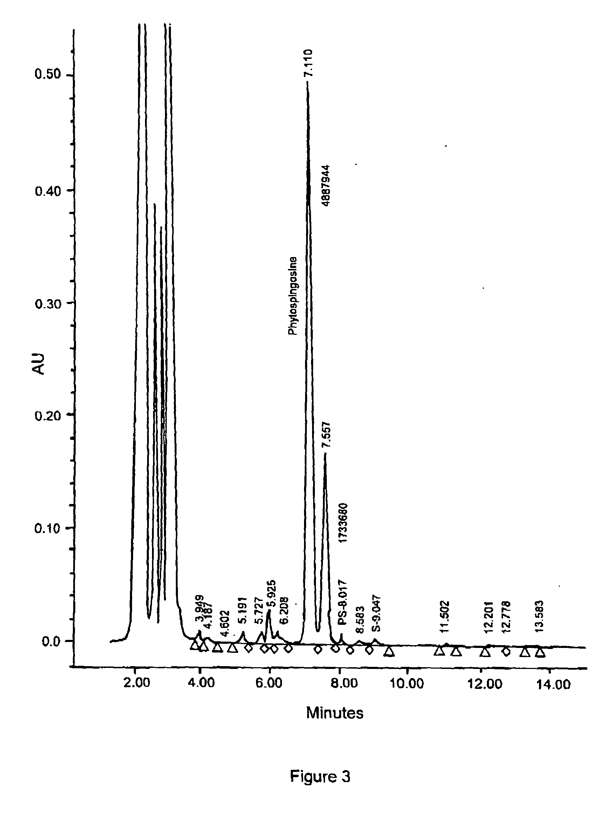

An UV-detector was used for the analysis of the acetic anhydride derivatives. phytosphingosine (PS) and sphingosine (So) were separated with retention times of 7.5 and 7.1 minutes, respectively (see Figure 3). The detection limit was determined at: 20 µM PS.

### EXAMPLE 2

### Analysis of sphingoid bases via derivatization with trifluoro acetic anhydride

As a derivatization reagent 600 µl trifluoro acetic anhydride was mixed with 40 mg 4-(dimethylamino) pyridin and 200 µl triethylamine. Chloroform was added till 10 ml and the solution was mixed on a magnetic stirrer. Two mg of either phytosphingosine or sphingosine were mixed with 250 µl derivatization reagent and sonicated until the crystals are dissolved. The solutions were placed in a 35°C water bath for 30 min, acetonitrile was added up to 5 ml and mixed with a vortex. Ten µl was analysed by HPLC using conditions of Example 1.

An UV-detector was used for the analysis of the acetic anhydride derivatives. PS and So were well separated with retention times of 10.1 and 8.9 minutes, respectively . The detection limit was determined at: 10 µM PS.

### EXAMPLE 3

### Analysis of sphingoid bases via derivatization with o-phthalaldehyde

As a derivatization reagent 50 mg OPA (o-phthalaldehyde) was dissolved in 1 ml ethanol and 50 µl 2-mercaptoethanol. Subsequently 99 ml 3% boric acid in water, pH 10,5 (with KOH) was added. Two mg of either phytosphingosine or sphingosine were mixed with 2.5 ml MeOH and sonicated until the crystals are dissolved. Then 2.5 ml OPA derivatization reagent was added, after mixing the samples were incubated for 15 minutes at room temperature. Before analysis with HPLC, the samples were cleared via centrifugation. The HPLC conditions were as follows.

| HPLC conditions | |
|---|---|
| Mobile phase | 90% (v/v) methanol in 0.5 mM potassium phosphate buffer pH 7.0 |
| Column | Radial Pak C18 column Waters (8 x 100 mm) |
| Flow | 2 ml/min |
| Fluorescence detector | |
| Excitation wavelength | 340 nm |
| Emission wavelength | 455 nm |

For detection a scanning fluorescence detector was used. OPA derivatives of PS and So were well separated with retention times of 5.1 and 8.0 minutes, respectively. The detection limit was determined at: 2 µM PS

### EXAMPLE 4

To find organisms capable of producing sphingosine the sphingoid base substrates, which are very hydrophobic compounds, need to be in contact with the necessary enzymes in their right environment. Therefore, cells of different species (see Table 1) were grown to mid-exponential phase, washed and incubated with 0.5% toluene for half an hour while gently shaking (10 to 100 rpm) during the whole incubation period. Cells were washed again and tested for permeability by performing an internal enzyme assay according to methods commonly known to the person skilled in the art.. A suitable enzyme is isocitrate dehydrogenase. A portion of permeabilized cells is incubated with the necessary substrates and co-factors, incubated at the appropriate temperature and cofactor/product formation is analysed afterwards.

### EXAMPLE 5

To evaluate the recovery of sphingoid bases from cell matrices, permeabilised cells were incubated with 300 µM of phytosphingosine for 2 hours. Afterwards, samples were spun down to remove excess phytosphingosine. Cells were subsequently resuspended in 50 mM TRIS-HCl pH 7.4, frozen and freeze-dried overnight. After lyophilization the sphingoid bases were extracted by addition of pure methanol and periodically vortexing for half an hour. The supernatant obtained after centrifugation was derivatized according to the o-phthalaldehyde method (see Example 3) and the average recovery was at least 71% with high reproducibility (see Table 1).

**Table 1. Results from phytosphingosine recovery**

| **Microorganism** | **Recovery phytosphingosine (%)** |
|---|---|
| *Acinetobacter calcoaceticus* | 82 4 |
| *Escherichia coli* | 75 8 |
| *Mycobacterium neoaurum* | 90 1 |
| *Ochrobactrum anthropi* | 71 1 |
| *Pseudomonas putida* | 82 4 |
| *Rhodococcus erythropolis* | 87 7 |
| *Saccharomyces cerevisiae* | 75 1 |

### EXAMPLE 6

### Protocol for incubation of biological samples with phytosphingosine

1. Pre-grow the organisms in 10 ml medium in a 50 ml shake flask.
Bacteria for ~24-36 hours; fungi for ~72-96 hours.
2. Dilute 100x fold by transferring 200 µl of the pre-grown culture in to 20 ml fresh medium in 100 ml shake flasks (for fungi the amount to be transferred may be adapted if they grow in pellets or to slow) and grow to exponential phase (if Optical Density measurements are applied then up to an OD₆₀₀ₙₘ of ~4-5).
For bacteria ~6-8 hours; for fungi ~24-72 hours (depending on the growth rate of the fungi).
3. Harvest cells by centrifugation.
4. Resuspend the pellet in 2.5 ml of Tris/HCl (50 mM; pH 7.4) in 12 ml test tubes (glass or plastic).
5. Add 2.5 ml of 1% toluene in Tris/HCl (50 mM; pH 7.4). 1% toluene does not dissolve in the buffer; therefore the mixture must be stirred vigorously.
6. Incubate for 10 minutes on a magnetic stirrer with 1200 rpm at the same temperature as the applied growth temperature in steps 1 and 2.
7. Harvest cells by centrifugation.
8. Resuspend the pellet in 10 ml Tris/HCl (50 mM; pH 7.4).
9. Harvest cells by centrifugation.
10. Resuspend the pellet in 2 ml Tris/HCl (50 mM; pH 7.4). The permeabilized cells can be used immediately or may be stored at -70°C.
11. Add 71 µl of Phytosphingosine stock solution to the 1 ml of permeabilized cells.
*Stock solution Phytosphingosine:*
440 mg phytosphingosine (free base)
8 ml 20 % (v/v) Tween-80 in milliQ (=de-ionized water)
2 ml 96% ethanol.
Adjust pH with HCl until Phytosphingosine is dissolved (pH ~5.8)
Final concentration of stock solution is 140 mM
12. Incubate for 2 hours with shaking (100 rpm) and apply same temperature as used for growth in step 1 and 2.
13. Store the samples at -70°C until you have enough samples for lyophilization.
Lyophilise samples in the 12 ml plastic test tube overnight.
14. Without any additions pulverize the residue using a vortex shaker.
15. Add 2.5 ml Trifluoroacetic anhydride derivatization reagent.
*Trifluoroacetic anhydride derivatization reagent (for 100 reactions) :*
2,0 g 4-(dimethylamino) pyridin
30 ml Trifluoroacetic anhydride
10 ml triethylamine
Fill up to 500 ml with chloroform and mix the solution with a magnetic stirrer.
The solution is stable for one month, stored at 4°C
16. Vortex vigorously to obtain an homogenous mixture.
17. Apply ultrasonic treatment to remove any crystals.
18. Incubate the mixture for 30 minutes in a water bath at 35°C .
19. Centrifuge the mixture.
20. Transfer the supernatant to a new tube.
21. Store supernatant at less then 8°C and analyse within 48 hours due to the stability of the derivatized material.
22. Prepare derivatized standards of phytosphingosine and sphingosine fresh each time.
23. Phytosphingosine standard:
2 mg phytosphingosine
add 250 µl Trifluoroacetic anhydride derivatization reagent
apply ultrasonic treatment to remove any crystals
*Trifluoroacetic anhydride derivatization reagent (for 40 standard solutions):*
40 mg 4-(dimethylamino) pyridin
600 µl Trifluoroacetic anhydride
200 µl triethylamine
Fill up to 10 ml with chloroform and mix the solution with a magnetic stirrer.
The derivatization solution is stable for one month, stored at 4°C
24. Sphingosine standard:
1 mg sphingosine
add 250 µl Trifluoroacetic anhydride derivatization reagent
apply ultrasonic treatment to remove any crystals
25. Incubate both mixtures for 30 minutes in a water bath at 35°C.
26. add 1750 µl acetonitrile.
27. mix by vortexing.
28. centrifuge.
29. use 10 µl of the supernatant for HPLC analysis.
30. vortex and centrifuge samples from step 20.
31. use 10 µl of the supernatant for HPLC analysis.
32. HPLC conditions:

| | | |
|---|---|---|
| Mobile phase | 0.5 ml Trifluoroacetic acid in 1000 ml acetonitrile | |
| Column | GL Science, Inertsil ODS 80-A, 4.6 x 250 mm | |
| Flow | 1 ml/min | |
| UV-detection | at 200 nm | |
| RetentionTime | Phytosphingosine: | 10.1 minutes |
| RetentionTime | Sphingosine: | 8.9 minutes |

33. Each sample takes 15 minutes to be analysed. The sample is run trough (phytosphingosine and sphingosine come after 10.1 and 8.9 minutes) and the column is washed for the same time; there is no need for additional washing.

### EXAMPLE 7

### Sphingosine production by Pseudomonas

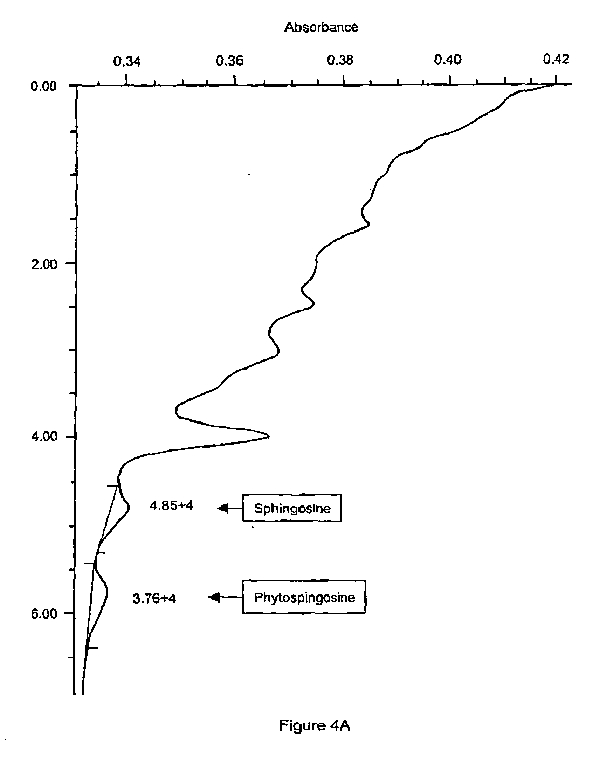

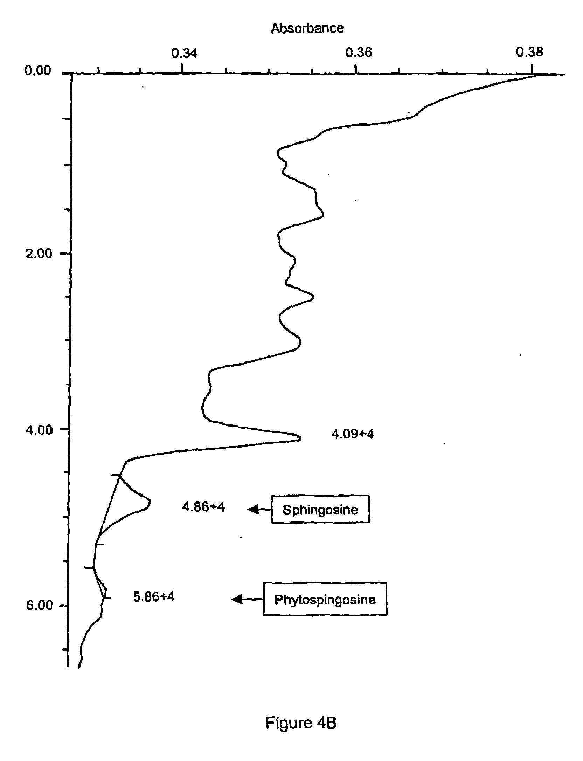

*Pseudomonas aurantiaca* (VKM B-1524) was precultivated on PDA agar plates (per liter: Potato extract, 230 ml; glucose, 20.0 g; agar, 20.0 gr; pH 6.0) for 4 days at 25 C. This was used to inoculate shake flasks with P-M medium (per liter: meat extract, 5.0 g; pepton, 10.0 g; yeast extract, 3.0 g; NaCl, 3.0 g; glucose, 10.0 g; KH₂PO₄, 3.0 g; MgSO₄ x 7H₂O, 5.0 g; pH 7.2) for 2 days at 25 C. The cells were processed as described in example 6 and 10-28% of the extracted sphingoid bases was sphingosine (see Figure 4), while no sphingosine could be detected in the control sample. To identify the formed products sphingosine was spiked to the sample and analysed again. Clearly the peak at 4.85/4.86 minutes is increased, confirming the formation of sphingosine (compare Figures 4A and 4B).

### EXAMPLE 8

### Sphingosine production by Grifola

*Grifola frondosa* (VKM F-3125) was precultivated on liquid malt with glass beads (per liter: Malt extract, 300 ml; pH 6.0) for 10 days at 25 C. This was used to inoculate (using 5% of the preculture) shake flasks with agaric medium (per liter: pepton, 2.0 g; yeast extract, 2.0 g; glucose, 20.0 g; K₂HPO₄, 1.0 g; KH₂PO₄, 0.5 g; MgSO₄ x 7H₂O, 0.2 g; pH 6.3-6.4) for 10 days at 25 C. The cells were processed as described in example 6 and 13% of the extracted sphingoid bases was sphingosine , while no sphingosine could be detected in the control sample.

### EXAMPLE 9

### Sphingosine production by Streptomyces

*Streptomyces* sp. (Ac-109) was precultivated on P-Y medium (per liter: pepton, 5.0 g; yeast extract, 3.0 g; glucose, 5.0 g; KH₂PO₄, 0.2 g; pH 7.0-7.2) for 3 days at 26 C. This was used to inoculate a second shake flask with P-Y medium using 5% of the preculture as an inoculum, for 3 days at 26 C. The cells were processed as described in example 6 and 19% of the extracted sphingoid bases was sphingosine, while no sphingosine could be detected in the control sample.

### EXAMPLE 10

### Sphingosine production by Inonotus

*Inonotus dryophilus* (VKM F-434) was precultivated on liquid malt with glass beads (per liter: Malt extract, 300 ml; pH 6.0) for 6 days at 25 C. This was used to inoculate (using 5% of the preculture) shake flasks with agaric medium (per liter: pepton, 2.0 g; yeast extract, 2.0 g; glucose, 20.0 g; K₂HPO₄, 1.0 g; KH₂PO₄, 0.5 g; MgSO₄ x 7H₂O, 0.2 g; pH 6.3-6.4) for 3 days at 25 C. The cells were processed as described in example 6 and 7% of the extracted sphingoid bases was sphingosine.

### EXAMPLE 11

### Sphingosine production by Hericium

*Hericium coralloides* (VKM F-3128) was precultivated on liquid malt with glass beads (per liter: Malt extract, 300 ml; pH 6.0) for 10 days at 25 C. This was used to inoculate (using 5% of the preculture) shake flasks with agaric medium (per liter: pepton, 2.0 g; yeast extract, 2.0 g; glucose, 20.0 g; K₂HPO₄, 1.0 g; KH₂PO₄, 0.5 g; MgSO₄ x 7H₂O, 0.2 g; pH 6.3-6.4) for 3 days at 25 C. The cells were processed as described in example 6 and 18% of the extracted sphingoid bases was sphingosine, while no sphingosine could be detected in the control sample.

### EXAMPLE 12

### Growth of Phytophthora

*Phytophthora capsici* (VKM F- 2062) was precultivated on V8 agar plates (per liter: V8 vegetable juice, 125 ml; CaCO₃ 2.5 g; agar 20 g). One square cm blocks were cut and used to inoculate 15 ml liquid medium in plastic petri dishes without shaking. Several media were used: Ph (per liter: yeast extract, 5.0 g;; glucose, 5.0 g; L-Asparagine, 1.0 g; KH₂PO₄, 0.5 g; MgSO₄ x 7H₂O, 1.0 g; thiamine, 0.001; pH 6.0), V8 and HV8 (same as V8, but after mixing of V8 vegetable juice and CaCO₃ the debris was removed). All media were supplemented with pimaricin (100 mg/l), rifampicin (10 mg/l), vancomycin (10 mg/ml) and ampicillin (100 mg/l). After 64 hours growth was clearly visible.

### EXAMPLE 13

### Sphingosine production by Phytophthora

*Phytophthora capsici* (VKM F- 2062) was precultivated on PDA agar plates (per liter: Potato extract, 230 ml; glucose, 20.0 g; agar, 20.0 gr; pH 6.0) for 14 days at 25 C. This was used to inoculate liquid Ph medium (per liter: yeast extract, 5.0 g;; glucose, 5.0 g; L-Asparagine, 1.0 g; KH₂PO₄, 0.5 g; MgSO₄ x 7H₂O, 1.0 g; thiamine, 0.001; pH 6.0) for 3 days at 25 C. The cells were processed as described in Example 6 and 29% of the extracted sphingoid bases was sphingosine, while no sphingosine could be detected in the control sample.

### EXAMPLE 14

### o-phthalaldehyde analysis of Pseudomonas and Tricellula sphingoid bases

*Tricellula aurantiaca* (VKM F-2456) and *Pseudomonas aurantiaca* (VKM B-1524) were cultivated as described in example 8. After freeze drying the samples were derivatised using o-phthalaldehyde, as described in example 3. Both species produced sphingosine after incubation with phytosphingosine.

### EXAMPLE 15

### Sphingosine production and NMR analysis by Pythium

*Pythium adhaerens* (VKM F-1921) was precultivated on malt agar petri dishes (per liter: Malt extract, 300 ml; agar, 20.0 g; pH 6.0) for 20 days at 25 C. Blocks with fungus were cut from these agar plates and used to inoculate (using 20 blocks of the preculture) liquid agaric medium (per liter: pepton, 2.0 g; yeast extract, 2.0 g; glucose, 20.0 g; K₂HPO₄, 1.0 g; KH₂PO₄, 0.5 g; MgSO₄ x 7H₂O, 0.2 g; pH 6.3-6.4) for 3 days at 25 C. The cells were processed as described in example 6, but analysed with Nuclear Magnetic Resonance (NMR) and sphingosine was observed.

## Claims

1. A process to identify a microorganism that is able to convert at least part of exogenously added phytosphingosine into sphingosine comprising:
providing a series of candidate microorganisms,
incubating said series of candidate microorganisms in the presence of a suitable amount of phytosphingosine,
analyzing the composition of the sphingoid base obtained from each incubated microorganism, and
identifying a microorganism that is able to convert at least part of the phytosphingosine into sphingosine.

2. A process according to claim 1, wherein the candidate microorganisms are permeabilised prior to incubation with phytosphingosine.

3. A process according to claim 1, wherein the phytosphingosine is solubilised in an aqueous solution.

4. A process according to claim 1, wherein the sphingoid base is obtained by extracting the sphingoid base from each incubated microorganism and derivatising the sphingoid base.

5. A process according to claim 4, wherein the derivatisation is done by treatment of the sphingoid base with acetic anhydride, trifluoro acetic anhydride or o-phthalaldehyde.

6. A process according to claim 1, wherein a suitable amount of phytosphingosine is 0.1 to 100 mM, preferably 1 to 20 mM.

7. A process according to claim 1, wherein the sphingosine content of the obtained sphingoid base is at least 5% (w/w), preferably at least 10% (w/w), more preferably at least 15% (w/w), most preferably at least 20% (w/w), using phytosphingosine in an amount of 10 mM.

8. A process according to claim 1, wherein the identified microorganism is from the genus *Pseudomonas*, *Grifola, Streptomyces, Inonotus, Hericium, Phytophthora, Tricellula,* and/or *Pythium.*

9. A process according to claim 1, wherein the identified microorganism is from the species *Pseudomonas aurantiaca, Grifola frondosa, Inonotus dryophilus, Hericium coralloides, Phytophthora capsici, Tricellula aurantiaca* and/or *Pythium adhaerens.*

10. A process for the manufacturing of sphingosine comprising cultivating a microorganism as identified in Figure 2, preferably a microorganism from the species *Streptomyces* sp, *Pseudomonas aurantiaca, Grifola frondosa, Inonotus dryophilus, Hericium coralloides, Phytophthora capsici, Tricellula aurantiaca* and/or *Pythium adhaerens,* incubating the microorganism in the presence of phytosphingosine to convert at least part of the phytosphingosine into sphingosine and recovering the sphingosine.

11. Use of a microorganism as identified in Figure 2, preferably a microorganism from the species *Streptomyces* sp, *Pseudomonas aurantiaca, Grifola frondosa, Inonotus dryophilus, Hericium coralloides, Phytophthora capsici, Tricellula aurantiaca* and/or *Pythium adhaerens,* to isolate the enzyme activity that converts phytosphingosine into sphingosine.

12. Use of a microorganism as identified in Figure 2, preferably a microorganism from the species *Streptomyces* sp, *Pseudomonas aurantiaca, Grifola frondosa, Inonotus dryophilus, Hericium coralloides, Phytophthora capsici, Tricellula aurantiaca* and/or *Pythium adhaerens,* to obtain a microorganism that produces sphingosine, by isolating the gene(s) encoding the enzyme activity that converts phytosphingosine into sphingosine, transforming the gene(s) into a suitable phytosphingosine-producing microorganism and isolating a transformed microorganism that produces sphingosine.

13. Use according to claims 11 or 12, wherein the enzyme is a dehydratase.

## Patentansprüche

1. Ein Verfahren zum Identifizieren eines Mikroorganismus, der in der Lage ist, mindestens einen Teil von exogen hinzugefügtem Phytosphingosin in Sphingosin umzuwandeln, das umfasst:
- Bereitstellen einer Reihe von Kandidatenmikroorganismen;
- Inkubieren der Reihe von Kandidatenmikroorganismen in der Gegenwart einer geeigneten Menge von Phytosphingosin;
- Analysieren der Zusammensetzung der sphingoiden Base, die von jedem inkubierten Mikroorganismus erhalten wird; und
- Identifizieren eines Mikroorganismus, der in der Lage ist, mindestens einen Teil des Phytosphingosins in Sphingosin umzuwandeln.

2. Ein Verfahren gemäß Anspruch 1, wobei die Kandidatenmikroorganismen permeabilisiert werden vor der Inkubation mit Phytosphingosin.

3. Ein Verfahren gemäß Anspruch 1, wobei das Phytosphingosin in einer wässrigen Lösung aufgelöst wird.

4. Ein Verfahren gemäß Anspruch 1, wobei die sphingoide Base durch Extrahieren der sphingoiden Base von jedem inkubierten Mikroorganismus und durch Derivatisieren der sphingoiden Base erhalten wird.

5. Ein Verfahren gemäß Anspruch 4, wobei die Derivatisierung durch Behandlung der sphingoiden Base mit Essigsäureanhydrid, Trifluoressigsäureanhydrid oder o-Phthalaldehyd durchgeführt wird.

6. Ein Verfahren gemäß Anspruch 1, wobei eine geeignete Menge des Phytosphingosins 0.1 bis 100 mM, bevorzugt 1 bis 20 mM ist.

7. Ein Verfahren gemäß Anspruch 1, wobei der Sphingosin-Gehalt der erhaltenen sphingoiden Base mindestens 5% (w/w), bevorzugt mindestens 10% (w/w), bevorzugter mindestens 15% (w/w), am bevorzugtesten mindestens 20% (w/w) ist, wobei Phytosphingosin in einer Menge von 10 mM verwendet wird.

8. Ein Verfahren gemäß Anspruch 1, wobei der identifizierte Mikroorganismus aus der Gattung *Pseudomonas, Grifola, Streptomyces, Inonotus, Hericium, Phytophthora, Tricellula* und/oder *Pythium* ist.

9. Ein Verfahren gemäß Anspruch 1, wobei der identifizierte Mikroorganismus aus der Gattung *Pseudomonas aurantiaca, Grifola frondosa, Inonotus dryophilus, Hericium coralloides, Phytophthora capsici, Tricellula aurantiaca* und/oder *Pythium adhaerens* ist.

10. Ein Verfahren zur Herstellung eines Sphingosins, das das Kultivieren eines Mikroorganismus wie in Figur 2 identifiziert, bevorzugt eines Mikroorganismus der Spezies *Streptomyces sp, Pseudomonas aurantiaca, Grifola frondosa, Inonotus dryophilus, Hericium coralloides, Phytophthora capsici, Tricellula aurantiaca* und/oder *Pythium adhaerens,* das Inkubieren des Mikroorganismus in der Gegenwart von Phytosphingosin, um mindestens einen Teil des Phytosphingosins in Sphingosin umzuwandeln, und das Rückgewinnen des Sphingosins umfasst.

11. Verwendung eines Mikroorganismus wie in Figur 2 identifiziert, bevorzugt eines Mikroorganismus der Spezies *Streptomyces sp, Pseudomonas aurantiaca, Grifola frondosa, Inonotus dryophilus, Hericium coralloides, Phytophthora capsici, Tricellula aurantiaca* und/oder *Pythium adhaerens,* um die Enzymaktivität zu isolieren, die Phytosphingosin in Sphingosin umwandelt.

12. Verwendung eines Mikroorganismus wie in Figur 2 identifiziert, bevorzugt eines Mikroorganismus der Spezies *Streptomyces* sp, *Pseudomonas aurantiaca, Grifola frondosa, Inonotus dryophilus, Hericium coralloides, Phytophthora capsici, Tricellula aurantiaca* und/oder *Pythium adhaerens,* um einen Mikroorganismus zu erhalten, der Sphingosin erzeugt, durch Isolieren des/der Gens/Gene, die für die Enzymaktivität kodieren, die Phytosphingosin in Sphingosin umwandelt, Transformieren des/der Gens/Gene in einen geeigneten Phytosphingosinerzeugenden Mikroorganismus und Isolieren eines transformierten Mikroorganismus, der Sphingosin erzeugt.

13. Verwendung gemäß den Ansprüchen 11 oder 12, wobei das Enzym eine Dehydratase ist.

## Revendications

1. Procédé d'identification d'un micro-organisme qui est capable de convertir au moins une partie de la phytosphingosine ajoutée de manière exogène en sphingosine comprenant :
la fourniture d'une série de micro-organismes candidats,
l'incubation de ladite série de micro-organismes candidats en présence d'une quantité appropriée de phytosphingosine,
l'analyse de la composition de la base sphingoïde obtenue à partir de chaque micro-organisme incubé, et
l'identification d'un micro-organisme qui est capable de convertir au moins une partie de la phytosphingosine en sphingosine.

2. Procédé selon la revendication 1, dans lequel les micro-organismes candidats sont perméabilisés avant l'incubation avec la phytosphingosine.

3. Procédé selon la revendication 1, dans lequel la phytosphingosine est solubilisée dans une solution aqueuse.

4. Procédé selon la revendication 1, dans lequel la base sphingoïde est obtenue par extraction de la base sphingoïde à partir de chaque micro-organisme incubé et dérivatisation de la base sphingoïde.

5. Procédé selon la revendication 4, dans lequel la dérivatisation est réalisée par traitement de la base sphingoïde avec de l'anhydride acétique, de l'anhydride trifluoroacétique ou de l'o-phtalaldéhyde.

6. Procédé selon la revendication 1, dans lequel une quantité appropriée de phytosphingosine est de 0,1 à 100 mM, de préférence 1 à 20 mM.

7. Procédé selon la revendication 1, dans lequel la teneur en sphingosine de la base sphingoïde obtenue est au moins de 5 % (p/p), de préférence au moins 10 % (p/p), de manière davantage préférée au moins 15 % (p/p), de manière préférée entre toutes au moins 20 % (p/p), en utilisant de la phytosphingosine dans une quantité de 10 mM.

8. Procédé selon la revendication 1, dans lequel le micro-organisme identifié est du genre *Pseudomonas, Grifola, Streptomyces, Inonotus, Hericium, Phytophthora, Tricellula* et/ou *Pythium.*

9. Procédé selon la revendication 1, dans lequel le micro-organisme identifié est de l'espèce *Pseudomonas aurantiaca, Grifola frondosa, Inonotus dryophilus, Hericium coralloides, Phytophthora capsici, Tricellula aurantiaca* et/ou *Pythium adhaerens.*

10. Procédé de fabrication de sphingosine comprenant la culture d'un micro-organisme tel qu'identifié sur la figure 2, de préférence un micro-organisme de l'espèce *Streptomyces sp., Pseudomonas aurantiaca, Grifola frondosa, Inonotus dryophilus, Hericium coralloides, Phytophthora capsici, Tricellula aurantiaca* et/ou *Pythium adhaerens,* l'incubation du micro-organisme en présence de phytosphingosine pour convertir au moins une partie de la phytosphingosine en sphingosine et la récupération de la sphingosine.

11. Utilisation d'un micro-organisme tel qu'identifié sur la figure 2, de préférence un micro-organisme de l'espèce *Streptomyces sp., Pseudomonas aurantiaca, Grifola frondosa, Inonotus dryophilus, Hericium coralloides, Phytophthora capsici, Tricellula aurantiaca* et/ou *Pythium adhaerens,* pour isoler l'activité enzymatique qui convertit la phytosphingosine en sphingosine.

12. Utilisation d'un micro-organisme tel qu'identifié sur la figure 2, de préférence un micro-organisme de l'espèce *Streptomyces sp., Pseudomonas aurantiaca, Grifola frondosa, Inonotus dryophilus, Hericium coralloides, Phytophthora capsici, Tricellula aurantiaca* et/ou *Pythium adhaerens,* pour obtenir un micro-organisme qui produit de la sphingosine, par l'isolement du/des gène(s) codant pour l'activité enzymatique qui convertit la phytosphingosine en sphingosine, la transformation du/des gène(s) dans un micro-organisme produisant de la phytosphingosine approprié et l'isolement d'un micro-organisme transformé qui produit de la sphingosine.

13. Utilisation selon la revendication 11 ou 12, dans lequel l'enzyme est une déshydratase.
